# EUROPEAN PATENT APPLICATION

(11) **EP 4 759 414 A1**
(43) Date of publication of application: **17.06.2026**
(21) Application number: 25221051.3
(22) Date of filing: 05.12.2025
(51) Int. Cl.: B01L 3/00

(54) **ACOUSTIC SEPARATION AND LYSING**

(30) Priority: 11.12.2024 US 202418977622
(71) Applicant: Instrumentation Laboratory Company, Bedford, MA 01730 (US)
(72) Inventor: KERIMO, Josef, Concord 01742 (US); WANG, Yu, Westford 01886 (ES); LU, Quansheng, 02458 Newton (US); SUN, Hoi-Cheong Steve, 01803 Burlington (US)
(74) Representative: ZBM Patents - Zea, Barlocci & Markvardsen

(57) **Abstract**

An example system includes a flow cell containing a fluidic channel configured to receive a sample, where the sample includes plasma and blood cells. and one or more acoustic transducers configured to output acoustic waves to the fluidic channel. The one or more acoustic transducers are configured (i) to output acoustic waves in a first frequency range to separate the plasma from the blood cells in the fluidic channel, and (ii) to output acoustic waves in a second frequency range to lyse the blood cells in the fluidic channel. The first frequency range includes higher frequencies than the second frequency range. A spectrometer is configured (i) to perform detection on a region of the fluidic channel containing the plasma separated from the blood cells, and (ii) to perform detection on a region of the fluidic channel where the blood cells have been lysed.

## Description

### TECHNICAL FIELD

This specification relates generally to systems and processes that use acoustic separation and lysing to obtain both CO-oximetry measurements and hemolysis or analyte measurements.

### BACKGROUND

A test sample may include particles and liquid. For example, a test sample may be blood or a blood product that includes plasma and red blood cells.

Acoustic waves can be applied to the test sample to separate the particles from liquid. The acoustic waves create one or more pressure nodes within the test sample, which correspond to regions of maximum and minimum pressure in the test sample. The particles move to the locations of minimum pressure, thereby concentrating the particles in some regions of the test sample and leaving other regions of the test sample completely or substantially devoid of particles.

CO-oximetry includes a process that measures concentrations of different hemoglobins and oxygen saturation of hemoglobin in a test sample. Oxygen saturation or "sO₂" of a blood sample corresponds to the proportion of functional hemoglobins with bound oxygen in the blood sample. Lysis is a process where the red blood cells are disrupted to facilitate the CO-oximetry measurement,

### SUMMARY

An example system includes a flow cell containing a fluidic channel configured to receive a sample, where the sample includes plasma and blood cells. and one or more acoustic transducers configured to output acoustic waves to the fluidic channel. The one or more acoustic transducers are configured (i) to output acoustic waves in a first frequency range to separate the plasma from the blood cells in the fluidic channel, and (ii) to output acoustic waves in a second frequency range to lyse the blood cells in the fluidic channel. The first frequency range includes higher frequencies than the second frequency range. A spectrometer is configured (i) to perform detection on a region of the fluidic channel containing the plasma separated from the blood cells, and (ii) to perform detection on a region of the fluidic channel where the blood cells have been lysed. The example system may include one or more of the following features, either alone or in combination.

The spectrometer may be an imaging spectrometer. The system may include an imaging camera to capture an image of the fluidic channel. The one or more acoustic transducers may be bound (e.g., physically coupled, directly or indirectly) to the flow cell.

The system may include a test instrument that includes the spectrometer. The test instrument may include a slot. The system may include a cartridge that includes the flow cell. The cartridge may be configured to move into, and out of, the slot of the test instrument. The one or more acoustic transducers may be bound to the flow cell and therefore part of the cartridge. The cartridge may be configured to move into, and out of, the slot of the test instrument. The one or more acoustic transducers may be configured to move to into physical coupling with the flow cell when the cartridge is fully in the slot of the test instrument.

The one or more acoustic transducers may be a single acoustic transducer configured to output acoustic waves in both the first frequency range and the second frequency range. The first frequency range may be in a range of 0.5 to 30 megahertz (MHz) or greater and the second frequency range may be in a range of 10 kilohertz (KHz) to 500KHz. The region of the fluidic where the plasma separated from the blood cells is detected and the region of the fluidic channel where lysing is detected may be different parts of the fluidic channel. The region of the fluidic where the plasma separated from the blood cells is detected and the region of the fluidic channel where lysing is detected may be the same part of the fluidic channel.

The fluidic channel may have a first dimension in a first region and a second dimension in a second region. The second dimension may be different from the first dimension. The dimension may be or include one or more of a channel width or a channel depth. The fluidic channel may have a curvature connecting the first region and the second region.

An interior of the flow cell defining the fluidic channel may include structures that extend from or into a surface of the interior. The structures may have a height or depth within a range of 20 nanometers (nm) to 10,000 nm. The structures may have a shape that is at least one of: pyramidal, cylindrical, rectangular, diamond, half-spherical, conical, flat-bottom tear-drop, or spiked.

An example method includes the following operations: controlling one or more acoustic transducers to output acoustic waves to a fluidic channel containing a sample that includes plasma and blood cells, where the one or more acoustic transducers are controlled to output the acoustic waves in a first frequency range to separate the plasma from the blood cells in the fluidic channel; obtaining information from a region of the fluidic channel containing the plasma separated from the blood cells; obtaining a hemolysis measurement for the region of the fluidic channel containing the plasma separated from the blood cells based on the information obtained by the imaging spectrometer from the region of the fluidic channel containing the plasma separated from the blood cells; controlling the one or more acoustic transducers to output acoustic waves to the fluidic channel after separating the plasma from the blood, where the one or more acoustic transducers are controlled to output acoustic waves in a second frequency range to lyse the blood cells in the fluidic channel, where the second frequency range includes lower frequencies than the first frequency range; obtaining information from a region of the fluidic channel where the blood cells have been lysed; and obtaining a co-oximetry measurement for the region of the fluidic channel where the blood cells have been lysed based on the information obtained by the imaging spectrometer from the region of the fluidic channel where the blood cells have been lysed. The example method may include one or more of the following features, either alone or in combination.

Obtaining the information from the region of the fluidic channel containing the plasma may include controlling an imaging system to obtain the information from the region of the fluidic channel containing the plasma. Obtaining the information from the region of the fluidic channel where the blood cells have been lysed may include controlling an imaging system to obtain the information from the region of the fluidic channel where the blood cells have been lysed.

A test instrument may include the one or more acoustic transducers and the imaging spectrometer. The test instrument may include a slot. A cartridge may include a flow cell containing the fluidic channel. The one or more acoustic transducers may be bound to the flow cell containing the fluidic channel. The method may include detecting full insertion of the cartridge into the slot; and controlling the one or more acoustic transducers to move toward the flow cell after full insertion of the cartridge into the slot.

The region of the fluidic from which information is obtained about separation and the region of the fluidic channel from which information is obtained about lysing may be different parts of the fluidic channel. The region of the fluidic from which information is obtained about separation and the region of the fluidic channel from which information is obtained about lysing may be the same part of the fluidic channel.

Any two or more of the features described in this specification, including in this summary section, may be combined to form implementations not specifically described in this specification.

At least part of the devices, systems, and processes described in this specification may be configured or controlled by executing, on one or more processing devices, instructions that are stored on one or more non-transitory machine-readable storage media. Examples of non-transitory machine-readable storage media include read-only memory, an optical disk drive, memory disk drive, and random access memory. At least part of the devices, systems, and processes described in this specification may be configured or controlled using a computing system comprised of one or more processing devices and memory storing instructions that are executable by the one or more processing devices to perform various control operations. The devices, systems, and processes described in this specification may be configured, for example, through design, construction, composition, arrangement, placement, programming, operation, activation, deactivation, and/or control.

The details of one or more implementations are set forth in the accompanying drawings and the following description. Other features and advantages will be apparent from the description and drawings, and from the claims.

### DESCRIPTION OF THE DRAWINGS

Fig. 1 is a perspective view of part of an example flow cell having a fluidic channel to contain test sample to be subjected to acoustic separation and acoustic lysing.
Fig. 2 is a block diagram of an example analyzer in which the example flow cell may be inserted to obtain both CO-oximetry measurements and hemolysis or analyte measurements.
Fig. 3 is a graphic (a), and magnified portions (b) and (c) thereof, showing example structures that may be contained in the fluidic channel of the flow cell.
Fig. 4 is a drawing showing an example interaction between blood cells and an example pyramidal structure in a fluidic channel of the flow cell.
Fig. 5 is a cross-sectional view (a) of the part of an example flow cell of Fig. 1 and an image (b) of cells and substantially-cell-free regions in the flow cell.
Fig. 6 is an image (a) of a flow cell containing, in its fluidic channel, separated cells and substantially-cell-free regions and an image (b) of a cross-section of the flow cell containing, in its fluidic channel, the separated cells and substantially cell-free regions.
Fig. 7 is a block diagram of components of an example imaging spectrometer system.
Fig. 8 is a block diagram of components of another example imaging spectrometer system.
Fig. 9 is a block diagram of components of another example imaging spectrometer system.
Fig. 10 is a flowchart showing operations included in an example process that uses acoustic separation and lysing to obtain both CO-oximetry measurements and hemolysis or analyte measurements.
Fig. 11, comprised of Figs. 11A and 11B, includes images and graphs depicting measurements of hemolysis, lipids, and bilirubin obtained using the systems and processes described herein.
Fig. 12, comprised of Figs. 12A and 12B, includes images and graphs depicting measurements of tHb, O₂Hb, HHB, MetHB, and COHb obtained using the systems and processes described herein.

Like reference numerals in different figures indicate like elements.

### DETAILED DESCRIPTION

Described herein are examples of systems, and processes for use therewith, configured to determine both CO-oximetry measurements and hemolysis measurements or analyte measurements from the same biological test sample ("test sample"). An example test sample may include a blood or a blood product, and contains liquid, such as plasma, in addition to particles, such as the red blood cells ("cells").

Hemolysis includes the rupture or destruction of red blood cells in a test sample. The example systems and processes use optical detection to identify, and to determine a measurement of, hemolysis in the test sample. The example systems and processes may also use optical detection to determine, for the test sample, the presence of and/or a measurement of analytes such as, but not limited to, glucose, lactate, sodium, potassium, chloride, phosphate, total protein, alanine aminotransferase, aspartate aminotransferase, alkaline phosphatase, lactate dehydrogenase, albumin, total globulin, hemoglobin, troponin I, cholesterol, coagulation factors, circulating tumor cells (CTC), and/or cell fractions.

CO-oximetry measurements include measurements of concentrations of different hemoglobins and oxygen saturation of hemoglobin in a test sample. Oxygen saturation or "sO₂" of a blood sample corresponds to the proportion of functional hemoglobins with bound oxygen in the blood sample. The example systems and processes may use optical detection to identify, and to determine CO-oximetry measurements of, one or more of the following: functional hemoglobin species including oxygen-carrying oxyhemoglobin (O2Hb) and de-oxyhemoglobin (HHb), or dysfunctional hemoglobin derivatives such as carboxyhemoglobin (COHb), methemoglobin (MetHb), sulfhemoglobin (SHb), and cyanmethemoglobin (CNmetHb). The example systems and processes may be used to determine CO-oximetry measurements of lipids, fetal hemoglobin, adult hemoglobin, and the concentration of bilirubin in a blood sample. Bilirubin is a hemoglobin degradation byproduct.

The example systems and processes are configured to apply acoustic waves in a first frequency range to the test sample to separate the test sample into one or more regions containing substantially cell-free plasma and one or more regions containing blood cells, such as red blood cells. In some implementations, substantially cell-free plasma includes no more than 10% by volume of red blood cells and in some implementations, substantially cell-free plasma includes no more than 1% by volume of red blood cells. In some implementations, platelets may be in the substantially cell-free regions. In some implementations, white blood cells may be in the substantially cell-free regions but they may be a very small percent by volume, e.g., less than 1%.

An optical detector, examples of which are described herein, obtains information from one or more cell-free plasma regions, from one or more blood cell regions, or from both cell-free plasma region(s) and blood cell region(s). The obtained information is used to determine the presence of hemolysis and/or one or more analytes in the sample and, in some cases, obtain measurements thereof.

The same systems and processes are also configured to apply, to the same test sample measured above, acoustic waves in a second frequency range, which is lower than the first frequency range, to lyse cells in the test sample. Lysing includes breaking down the cell membranes of the blood cells thereby releasing the lysate, including hemoglobin, from the cells. An optical detector, examples of which are described herein, obtains information from the lysate, which may be used to obtain CO-oximetry measurements from the test sample, such as measurements of concentrations of hemoglobin, bilirubin, lipids, and oxygen saturation in the lysed test sample.

The example systems and processes may be implemented on a clinical analyzer device that is usable in a point-of-care (POC) setting such as an emergency room. The clinical analyzer is an example of a test instrument or test system.

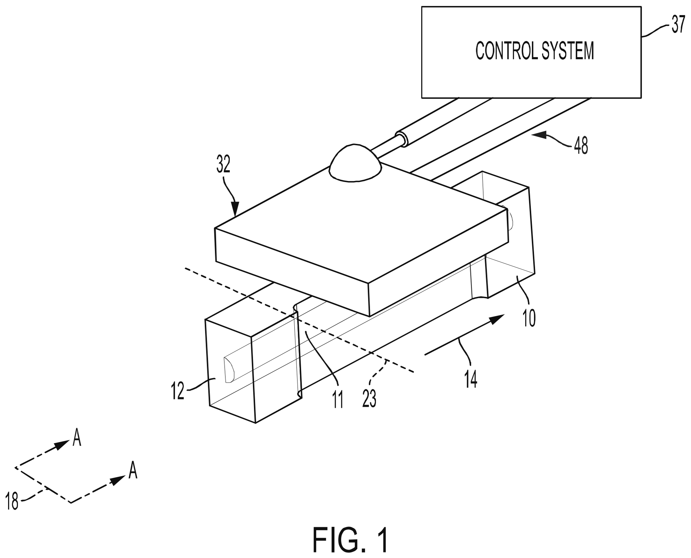

Fig. 1 is a perspective view of part of an example flow cell 10 that may be moved into, or out of, a clinical analyzer and that is configured to hold the test sample that is subjected to acoustic separation and lysing. An example of a test sample includes blood, such as whole blood, or a blood product. The test sample is comprised of particles such as red blood cells, white blood cells, platelets and lipid particles, and liquid such as substantially cell-free plasma. Flow cell 10 may be, or be part of a cartridge. that is configured to move into, and out of, the slot of a clinical analyzer.

Flow cell 10 includes a fluidic channel ("channel"), a portion of which is labeled 11. Channel 11 may be a microchannel. Flow cell 10 made of plastic, glass, or other wholly or at least partially transparent material, and may implement the microchannel as a conduit, a duct, a tube, or multiple interconnected conduits, ducts, or tubes configured to allow a fluid, such as a test sample described herein, to flow therethrough e.g., in the direction of arrow 14. In the examples described herein, the "cross-section" of the channel is across the width and depth of the channel; that is a view face-on of the width and depth of the channel. Channel 11 may have a semicircular cross-section (shown), a rectangular cross-section, a circular cross-section or any other shape that can sustain a standing wave and allow test sample to flow through the channel.

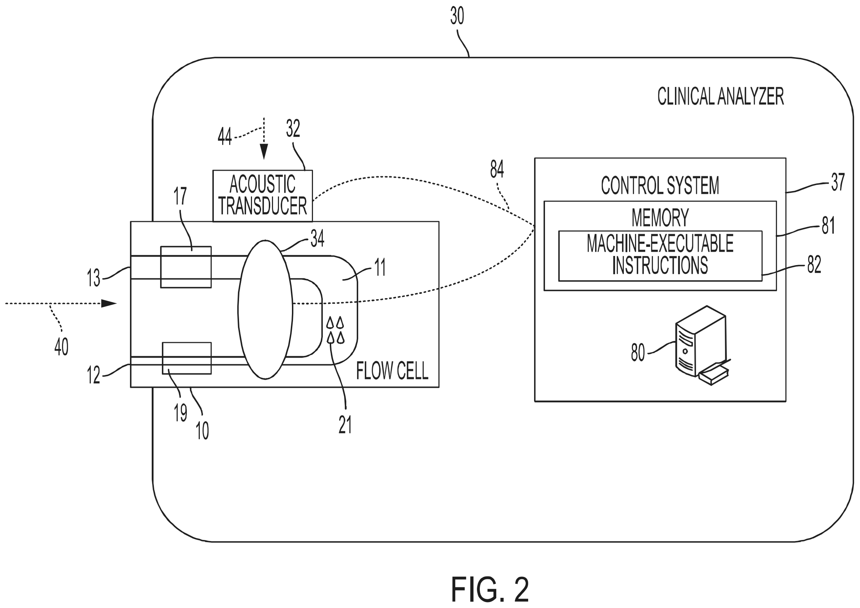

Channel 11 may be curved or U-shaped in this example, as shown in Fig. 2. In some cases, smaller channel widths promote cell/plasma separation whereas larger channel widths promotes lysing. For example, based on experiments conducted, channels having narrower widths separate blood more easily than channels having larger widths, while channels having larger widths facilitate blood lysing better than channels having narrower widths. Accordingly, channel 11 may have a wider channel width at region 17 where lysing occurs than at region 19 where cell/plasma separation occurs. To compensate for this difference in width, another dimension of the channel, such as a depth of the channel, may be larger at region 19 than at region 17.

In some implementations, the depth at region 17 is in a range including 0.1 mm to 0.13 mm. The depth at region 19 can be larger than the depth at region 17 (e.g., 0.32 mm). In some implementations, channel 11 at region 19 may have a width that is in a range of 0.1 mm to 1.64 mm (e.g., 0.82 mm). In another example, channel 11 at region 17 may have a width that is in a range 1.0 mm to 3 mm (e.g., 2 mm). In some implementations, the depth and width of channel 11 may be the same at regions 17 and 19 (not shown). In all instances other dimensions, such as channel width may be substituted for depth. Another example reason for having a deeper channel at region 19 than 17 is to get more signal from plasma for a hemolysis measurement, because hemolysis (e.g., free hemoglobin in plasma) at region 19 has a much lower concentration than the lysed blood (high concentration hemoglobin) at region 17.

In some implementations, the interior surface of the flow cell containing channel 11 is smooth. In some implementations, the interior surface of the flow cell containing channel 11 includes structures that extend from or into a surface of the interior of the channel. The structures add roughness or texture to the interior of the flow cell containing channel. In an example, the structures may constitute protrusions on the interior surface of the flow cell containing channel 11 that extend generally toward an interior (e.g., a center) of the channel. In an example, the structures may constitute indentations on the interior surface of the flow cell containing channel 11 that extend generally away from the interior of the channel. In some implementations, the structures may have a height or depth that exceeds 20 nanometers (nm). In some implementations, the structures may have a height or depth within a range of 20nm to 10,000nm. In some implementations, the structures may have a height or depth within a range of 625nm to 2500nm. In some implementations, the structures have a shape that is at least one of: pyramidal, cylindrical, rectangular, diamond, half-spherical, conical, flat-bottom tear-drop, or spiked. In some implementations, there may be 10 to 10,000 structures per square millimeter (mm²) of the interior surface of the flow cell containing the channel. In some implementations, there may be 100 to 500 structures (e.g., around 400 structures) per mm² of the surface of the interior of the flow cell containing the channel. In some implementations, the structures may extend along the entirety of the interior surface of the flow cell containing the channel. In some implementations, the interior surface of the flow cell containing a single channel may contain differently-shaped structures.

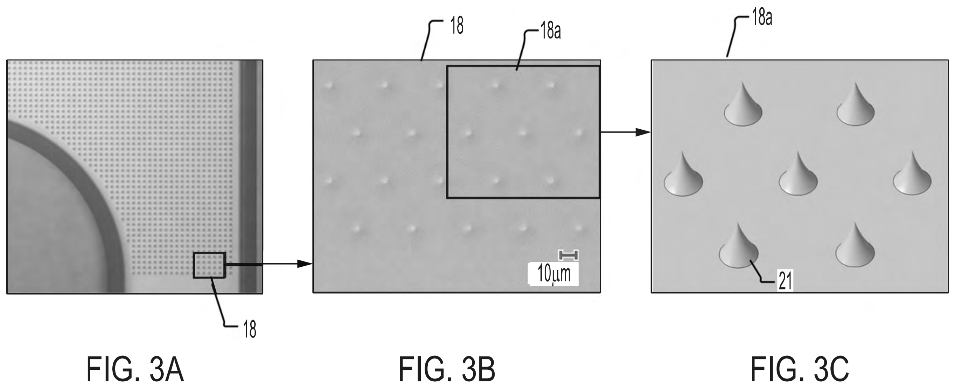

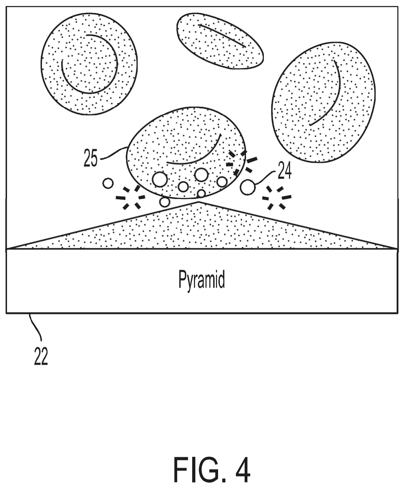

Fig. 3 shows examples of structures 21 of the type described above on an interior surface of the flow cell containing channel 11, with figure (a) showing a region 18 of the interior surface of the flow cell containing the channel to be magnified, figure (b) showing region 18 and a region thereof 18a to be magnified, and figure (c) showing region 18a magnified. Structures 21 are protrusions in this example. Examples of these structures 21 are also shown in Fig. 2, although not to scale and not in the quantity or concentration used or across the length of the cannel. The roughness or texture added to the interior of the flow cell containing of the channel by the structures support cavitation within the channel. That is, the structures serve as nucleation sites for cavitation of the test sample. Cavitation is a process in which the static pressure of a liquid reduces to below the liquid's vapor pressure, leading to the formation of small vapor-filled cavities in the liquid. Cavitation promotes - e.g., aids in - lysing of blood cells. This is illustrated in Fig. 4, which shows an example pyramidal structure 22 within a channel, such as channel 11. Vapor-filled cavities 24 produced through cavitation are around or proximate to pyramidal structure 22. Blood cells 25 within the channel to be lysed are also shown as being in contact with the vapor-filled cavities.

Referring back to Figs. 1 and 2, in this example, the test sample flows from an input location 12 of the flow cell through channel 11 to, and through, locations where separation, lysing, and detection are performed. For example, input location 12 may be fluidically connect to a fluid flow line that provides the sample to channel 11. Exit 13 may fluidically connect to a fluid flow line that accepts the sample exiting from channel 11. Positive or negative pressure applied to the channel and/or flow line by a pump (not shown) may cause the test sample to flow between the channel and the flow line.

Fig. 2 shows flow cell 10 installed in a clinical analyzer 30. In some implementations, acoustic transducer 32 is bound (e.g., physically coupled, directly or indirectly) to flow cell 10, as shown in Fig. 1. In some implementations, clinical analyzer 30 contains acoustic transducer 32, optical detector 34 (not shown in Fig. 1), and control system 37.

Acoustic transducer 32 may be a piezoelectric transducer. An example piezoelectric transducer includes a device that sends electrical energy in the form of an alternating electric field to a piezoelectric crystal in the transducer causing the crystal to vibrate and to convert the electrical energy into sound waves. Acoustic transducer 32 may be configured and controllable to generate one or more different types of acoustic waves, such as ultrasonic waves, surface acoustic waves, or bulk acoustic waves. In some implementations, the acoustic waves may have frequencies in the range of 10 kilohertz (KHz) to 30 megahertz (MHz) or greater or less than these values.

In some implementations, when in use for separation or lysing, the acoustic transducer is coupled to, e.g., in contact with, the flow cell containing channel 11. The contact may be direct or indirect. Indirect contact includes a substance or one or more structures between the acoustic transducer and the channel. For example, acoustic transducer 32 may be in contact with the flow cell through a coupler, such as ultrasound coupling gel or a solid state coupler. An example solid state coupler includes a low durometer elastomer that is thin and has an aperture to allow light transmission. An example durometer 15-60 may have a thickness within a range of 0.1mm to 1mm and may be made of materials such as silicone rubber (including PDMS - polydimethylsiloxane), polyurethane, elastomers, or the like. The coupler provides the benefit of a more reliable contact between the flow cell, such as glass, and the acoustic transducer, which may contain metal. The coupler also may provide thermal isolation between the flow cell and the acoustic transducer.

In some implementations the acoustic transducer is permanently bound to the flow cell. For example, the acoustic transducer is bound to the flow cell using an adhesive, which can serve as the coupler described above, or welding/melting at the interface between a surface of the transducer and a surface of the flow cell. In some implementations, a coupler as described above can be used where the coupler is permanently bound to both the acoustic transducer and the flow cell.

Acoustic transducer 32 may be excited using excitation parameters to produce acoustic waves. A control system, such as control system 37 described below, may provide the excitation parameters to acoustic transducer 32, or generate control signals based on the excitation parameters, to cause the acoustic transducer to generate acoustic waves having a specified amplitude, frequency, and/or duration. These excitation parameters include voltage/amplitude, energy/frequency, and timing/duration. A magnitude of the voltage applied to the acoustic transducer corresponds to the amplitude of the acoustic waves produced by the acoustic transducer. For example, the greater the voltage that is applied to the acoustic transducer, the greater are the amplitudes of the acoustic waves generated by the acoustic transducer. The frequency is the frequency of acoustic waves to be produced by the acoustic transducer. The frequency may be based on electrical energy provided to the acoustic transducer. The excitation parameters may include the magnitude and frequency of this electrical energy. The duration is the length of time that the acoustic waves are applied to the acoustic transducer. For example, the duration specifies the amount of time that the electrical energy and voltage are to be applied to the acoustic transducer to effect to a predefined amount (e.g., an optimal) separation of plasma and blood cells.

Referring to Fig. 2, in implementations where the acoustic transducer is in the clinical analyzer (as opposed to bound to the flow cell), acoustic transducer 32 is controllable to move towards, or into contact with, a flow cell 10 upon insertion of the flow cell into a clinical analyzer. This movement is shown with respect to example acoustic transducer 32. In this example, flow cell 10 is movable into clinical analyzer 30 in the direction of arrow 40. The movement may be implemented manually - for example, a clinician may insert the flow cell into the clinical analyzer. Control system 37 may be configured to cause acoustic transducer 32 to move toward, and into contact with, flow cell 10. For example, motors (not shown) may be used to control movement of the acoustic transducer in the direction of arrow 44 based on instructions from a control system. In some implementations, the transducer may be moved manually.

Control system 37 may be configured to make electrical contact with the acoustic transducer through electric wires 48 shown in Fig. 1. More specifically in the example of Fig. 1, electric wires 48, such as flexible cables, are soldered on the transducer and the other side of the wires connect to a circuit board of the control system. It is through this connection that the control system communicates with the acoustic transducer.

In some implementations, the flow cell, including one or more acoustic transducers bound thereto, is part of a cartridge that is inserted into the clinical analyzer. Accordingly, when the clinician inserts the cartridge into the clinical analyzer, acoustic transducer 32 bound to the flow cell is also inserted into the clinical analyzer. The movement may be aided by a roller or suction that are triggered upon insertion of cartridge into clinical analyzer 30. For example, control system 37 may be configured to detect that cartridge is fully inserted based on a part of the cartridge reaching a predefined location within clinical analyzer 30. Control system 37 may be configured to make electrical contact with the acoustic transducer through electrical contact(s), such as pad(s) or connector(s), between the control system and the acoustic transducer made during insertion of the cartridge into the clinical analyzer.

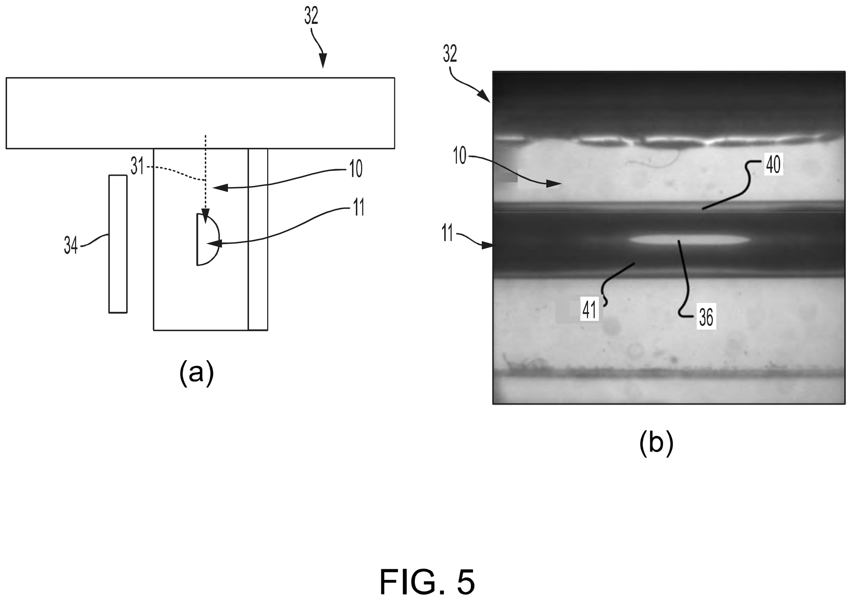

Fig. 5, in (a), shows flow cell 10 viewed along line A-A 18 of Fig. 1 and, in (b), shows an image of test sample in a cross-section of channel 11 taken along line 23 of Fig. 1. In Fig. 5, acoustic transducer 32 is configured and arranged to emit acoustic waves 31 that separate plasma from cells in channel 11 to form a region 36 of substantially cell-free plasma in a center of channel 11 and regions 40, 41 of red blood cells. Examples of regions of cell-free plasma includes layers of substantially cell-free plasma in the channel; and examples of regions containing blood cells include layers of red blood cells within the channel. These regions may also be referred to as particle layers and substantially particle-free layers.

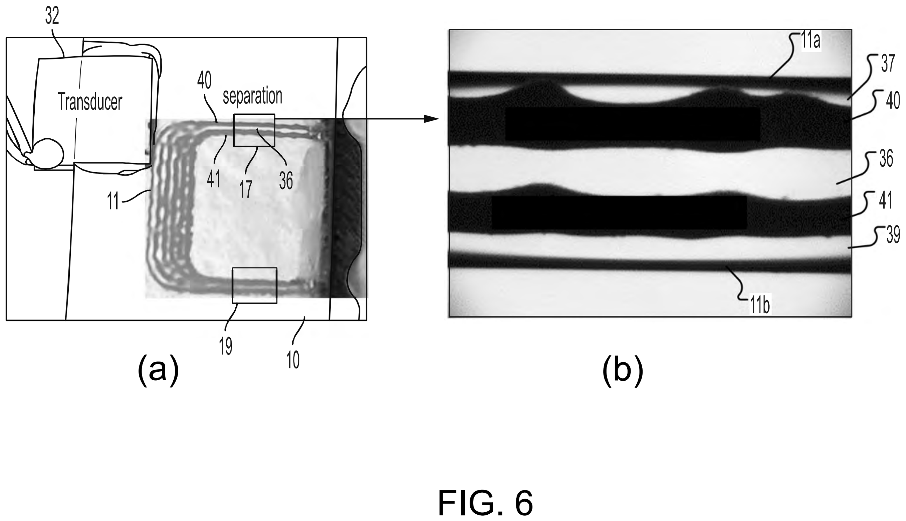

Fig. 6, in (a), shows transducer 32 positioned proximate to channel 11, with the test sample in channel 11 separated into regions 40, 41 containing cells and region 36 that is substantially cell-free. Fig. 6, in (b), shows an image of a cross-section of channel 11, with regions 40, 41 containing cells and region 36 that is substantially cell-free. As shown in Fig. 6(b), in some implementations, the acoustic waves from transducer 32 may also form substantially cell-free regions 37, 39 adjacent to one or more surfaces 11a, 11b of the flow cell defining channel 11.

In some implementations, there may be multiple cell and substantially cell-free regions (or layers) between the center and the surface 11a. 11b defining channel 11. In some implementations, regions 37, 39 may be absent, and the blood cells may abut and physically contact one or more surfaces 11a, 11b. In some implementations, there may be a region 36 of cell-free plasma in a center of the channel adjacent to regions 40, 41 containing blood cells, but with multiple additional regions of cell-free plasma and red blood cells between each region 40, 41 and surfaces 11a, 11b.

The examples of described herein illustrate separating the blood from the plasma such that at least the center of the channel (e.g., region 36 of Fig. 5(b)) contains substantially cell-free plasma. In some implementations, the blood cells and plasma may be separated such that the blood cells are in the center of the channel and plasma layers are between the blood cells in the center of the channel and a surface of the channel. For example, acoustic transducer 32 may be controlled by the control system to generate acoustic waves that produce pressure nodes within the channel such that the blood cells aggregate at the center of the channel and plasma layers are between the blood cells in the center of the channel and a surface of the channel. Following separation of this type, lysing may be performed as described herein.

Acoustic transducer 32 is also configured and controllable to generate acoustic waves in a lower frequency range than for separating cells and plasma. Such lower-frequency acoustic waves generated by acoustic transducer 32 are used to lyse blood cells in the test sample, rather than separating them from plasma. The lysed blood cells release lysate that mixes with the plasma already in the test sample. In some implementations, the lower-frequency acoustic waves generated by acoustic transducer 32 may have frequencies in the range of 10 to 500 KHz, such as 10 to 100KHz, 20 to 65KHz, 20 to 60 KHz or greater or less than these values. As described above, a control system, such as control system 37 described below, may provide excitation parameters to acoustic transducer 32, or generate control signals based on the excitation parameters, to cause the acoustic transducer to generate acoustic waves having a specified amplitude, frequency, and/or duration to lyse the blood cells in the test sample.

Referring to Figs. 2 and 5(a), one or more optical detectors are configured and arranged to perform optical detection on a region (e.g., region 19 or proximate thereto) of channel 11 containing the plasma separated from the blood cells, and to perform optical detection on a region (e.g., region 17 or proximate thereto) of channel 11 where the blood cells have been lysed. A single optical detector 34 may be used to perform detection on regions of channel 11 containing the plasma separated from the blood cells, and on regions of the channel 11 where the blood cells have been lysed.

The detection area for optical detector 34 may be placed at any location relative to channel 11 that enables the optical detector to perform detection at a part of the channel where the cells and plasma are separated, and where the test sample is lysed, at sufficient resolution. The distance between channel 11 and an optical detector 34, can be within 0 mm to 500 mm, depending on the system design (e.g. using lens(es) in between the channel and the camera or not). A light source (not shown in Figs. 2 and 5(a)), such as one or more light-emitting diodes (LEDs), may output light to illuminate channel 11 to enable the optical detectors to perform detection.

Referring to Fig. 6(a), for example, the cell/plasma separation and lysing may occur across the entirety of channel 11, although at different locations. However, regions 19 and 17, which have dimensions that promote separation and lysing, respectively, may be a focus for the detection by the optical detector described above.

Examples of types of optical detectors that may be used alone or in combination include, but are not limited to, a spectrometer, an imaging spectrometer, a camera, or a photometer. An imaging spectrometer differs from a conventional spectrometer in that it uses a two-dimensional (2D) detector array rather than a one-dimensional (1D) detector array. Furthermore, in some examples, aberrations of the internal optics may be optimized to allow a near-perfect imaging of an input slit onto the 2D detector array. The imaging spectrometer allows spectra from a less-than 10 mm long slit to be analyzed with almost tens of micrometers spatial resolution. Essentially the imaging spectrometer projects a line on the test sample and each pixel on that line can be interrogated separately. This means that it may be possible to measure the spectra of multiple regions along a line on a test sample simultaneously, and with tens of microns of spatial resolution.

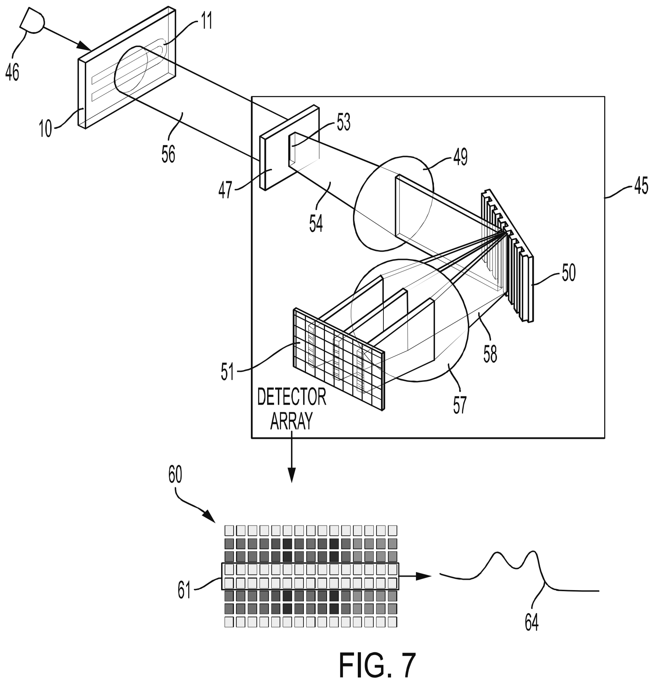

Fig. 7 is a block diagram of components of an example imaging spectrometer 45, which may be used as an optical detector 34. Example imaging spectrometer 45 operates by passing light from different parts of flow cell 10 to be imaged through a slit. A collimator collimates the light. The collimated light is dispersed by a diffraction grating into its component wavelengths - e.g., red, green, and blue light - and the component wavelengths are provided to a detector array such as a camera. The detector array obtains data relating to the spectral content of the light and generates an image based on that data. Portions of the resulting image are based on the spectral content of light from corresponding portions of the object that passed through the slit.

In the configuration shown in Fig. 7, imaging spectrometer 45 is configured to receive light from an LED 46 that passes through a portion of a channel 11 on example flow cell 10. Imaging spectrometer 45 includes a plate 47, lens(es) or mirror(s) 49, a diffraction grating 50, and a detector array 51. Plate 47 includes a slit 53 that allows a portion 54 of light 56 that passed through channel 11 to pass to optics, such as lens or mirror 49. Lens/mirror 49 is configured to collimate light 54 and to pass the resulting column of light to diffraction grating 50. Diffraction grating 50 is configured to disperse the light into its components 58, e.g., red light, green light, and blue light, and to direct those components to detector array 51, in this example via lens 57. Detector array 51, such as a camera, is configured to generate an image 60 based on the components. Each portion 61 of the image 60 corresponds to the spectral content 64 of light 54 that passed through slit 53. In addition, the imaging spectrometer may be controlled so that light from different portions of flow cell can be imaged and passed through slit 53, such as using a translation stage to move the flow cell or spectrometer, or using a scan mirror. Optics in the imaging spectrometer may include one or more mirrors and/or one or more lenses such as those shown configured to direct light.

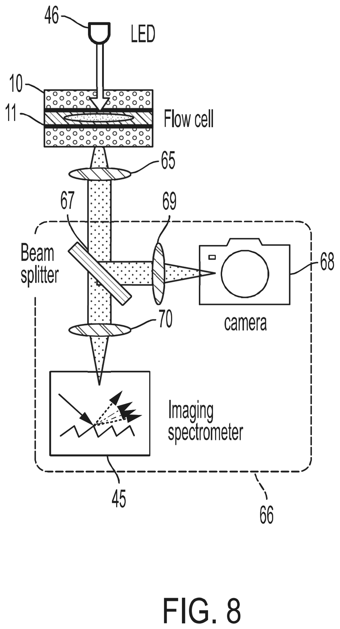

Fig. 8 is a block diagram of components of an example system 66, which may be an implementation of optical detector 34. System 66 is configured to receive light from LED 46 that passes through a portion of a channel 11 in an example flow cell 10. These components may be the same in structure and function as the same components shown in Fig. 7. The light may be collimated by optics, such as lens 65 and output to system 66. System 66 includes imaging spectrometer 45, which may be the same in structure and function as that shown in Fig. 7.

System 66 also includes a beam splitter 67, a camera 68, and optics such as lenses 69, 70. Beam splitter 67 is in the optical path between flow cell 10 and imaging spectrometer 45. Beam splitter 67 is configured to provide the light that passed through the channel of flow cell 10 to camera 68. The light may pass through optional focusing lens 69. Camera 68 is configured to generate a two-dimensional image of the channel. Beam splitter 67 is configured also to provide the light that passed through the channel of flow cell 10 to imaging spectrometer 45. The light may pass through optional focusing lens 70 before reaching imaging spectrometer 45.

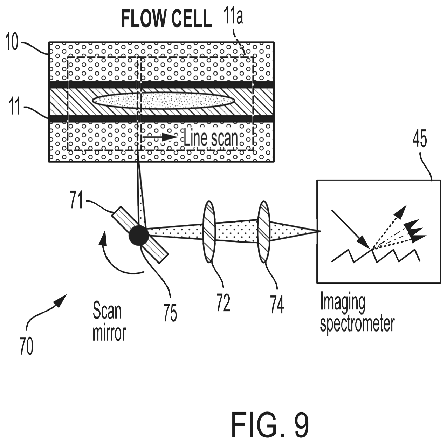

Fig. 9 is a block diagram of components of an example system 70, which may be an implementation of optical detector 34. System 70 is configured to receive light from an LED (not shown) that passes through a portion 11a of a channel 11 on an example flow cell 10. These components may be the same in structure and function as the same components shown in Figs. 7 and 8. System 70 includes imaging spectrometer 45, which may be the same in structure and function as the imaging spectrometer shown in Figs. 7 and 8.

System 70 also includes a scan mirror 71 and optics, such as lenses 72 and 74 in an optical path between flow cell 10 and imaging spectrometer 45. Scan mirror 71 is configured rotate about axis 75, which points out of the page, to scan the portion 11a of channel 11 on flow cell 10 to be imaged using imaging spectrometer 45. Scanning, in this context, includes receiving light from different portions of the channel / flow cell. A motor (not shown) may be used to control the rotation of scan mirror 71 based on instructions from the control system. In this example, lens 72 may collimate light from the scan mirror and lens 74 may focus light from lens 72 onto imaging spectrometer 45. Lenses 72 and 74 may be one lens instead or not needed depending on the optical system configuration. Imaging spectrometer 45 generates an image of the portion of the flow cell, as described with respect to Fig. 7. The movement of scanning mirror 71 allows the channel of the flow cell to be imaged without having to move the imaging spectrometer and/or the flow cell to capture a region of interest of the channel.

Referring back to Fig. 2, clinical analyzer 30 includes control system 37. The control system may be an internal component of clinical analyzer 30 or the control system may be external to clinical analyzer 30. For example, the control system may be or include one or more computing devices that are local to (e.g., within the same room as) or remote from (e.g., in a different room from) the clinical analyzer.

In this example, control system 37 may include one or more processing devices 80 and memory 81 storing instructions 82 that are executable. The one or more processing devices 80 may execute instructions 82 to control, or to implement at least part of, process 100 described below. Control system 37 is configured to communicate with a light source, such as LED 46, acoustic transducer 32, and optical detector 34, and components thereof over one or more data buses or other wired connections and/or over a wired or wireless computer network. Dashed lines 84 represent connections for some of these communication. These communications may include sending control signals from the various components and receiving information, such as information from the optical detector including spectral information and/or images of a portion of a channel that is being subjected to optical detection.

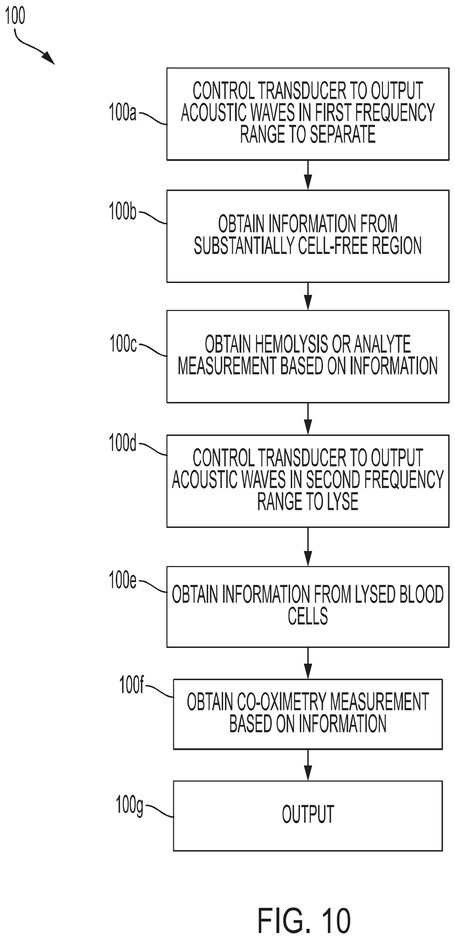

Fig. 10 shows operations included an example process 100 configured to use acoustic separation and lysing to obtain both hemolysis or analyte measurements and CO-oximetry measurements. Process 100 may be performed using the example systems or components thereof described with respect to Figs. 1 to 9 following introduction of a test sample into channel 11 of flow cell 10.

Process 100 includes controlling (100a) an acoustic transducer, such as acoustic transducer 32, to output acoustic waves in a first frequency range, such as in the range of megahertz range to 2GHz or greater, that cause separation of blood cells and plasma including at region 19 of channel 11. Controlling the acoustic transducer may include selecting values of excitation parameters for the acoustic transducer that produce a frequency in the target range, and instructing the transducer to operate using those values.

The acoustic waves cause the red blood cells and plasma to separate, as described herein, thereby producing a substantially cell-free plasma region 36 at a center of the channel as shown in Figs. 5(b) and 6(b) (or a cell region, not shown). This example uses the substantially cell-free plasma region 36 at the center of the channel, as noted above. In this example, substantially cell-free plasma region 36 has a separation width of 70 micrometers (µm) or more, where the separation width includes distance between two cell layers separated by the substantially cell-free (e.g. plasma) region at the center. For example, at its widest, cell-free region 36 may have a separation width of 800 µm.

Optical detector 34 obtains (100b) information from the substantially cell-free plasma region 36, e.g., by capturing one or more images of the substantially cell-free plasma region using, e.g., an imaging spectrometer, a camera, or both. Information coming from region 19 of channel 11 may be of a resolution or type that is most useful for separations-based measurements; however, optical detector 34 may obtain information from all regions of the channel that it covers as part of the detection process.

Control system 37 receives the information from optical detector 34. Control system 37 obtains (100c) a hemolysis or analyte measurement of the test sample by processing the images. For example, control system 37 may inspect the images to estimate plasma absorbance to detect the presence of, and to quantify, cell-free hemoglobin, which represents hemolysis. For example, control system 37 may inspect the images to identify attributes of one or more of the analytes described herein. Following separation and after acoustic transducer 32 is turned-off, the red blood cells and plasma may recombine, e.g., at any location where acoustic waves were applied and/or upstream and/or downstream of that location. In addition, one or more images of a calibration sample, such as a water or buffer solution, can be included to process the sample images.

Process 100 includes controlling (100d) the acoustic transducer, such as acoustic transducer 32, to output acoustic waves in a second frequency range, such 10KHz to 500KHz (e.g., 40KHz to 65KHz), that lyse the cells in the channel following separation including at region 17. As explained above, controlling the acoustic transducer may include the control system selecting values of excitation parameters for the acoustic transducer that produce a frequency in the target range, and instructing the transducer to operate using those values.

The acoustic waves cause the red blood cells to lyse, thereby releasing lysate that mixes with plasma in the test sample. Optical detector 34 obtains (100e) information about the lysate from the channel, e.g., by capturing one or more images of the channel using, e.g., an imaging spectrometer, a camera, or both. Information coming from region 17 of channel 11 may be of a resolution or type that is most useful for lysing-based measurements; however, optical detector 34 may obtain information from all regions of the channel that it covers as part of the detection process.

Control system 37 receives the information from optical detector 34. Control system 37 obtains (100f) a CO-oximetry measurement of the test sample by processing the images. For example, control system 37 may inspect the images to estimate plasma absorbance to detect the presence of, and to quantify, different hemoglobins and oxygen saturation of hemoglobin in the lysed test sample in the channel.

The presence and/or measurements of hemolysis or analyte, and the CO-oximetry measurements, may be output (100g) from the clinical analyzer on a user interface, either graphically or textually, and/or used in other diagnostic processes by the clinical analyzer.

The cell/plasma separation and lysing may be performed in the same region of the channel or in different regions of the channel. In some implementations, the cell/plasma separation described herein, including with respect to operation 100a, may be performed while the test sample is flowing through the fluidic channel. In some implementations, the lysing described herein, including with respect to operation 100d, may be performed while the test sample is flowing through the fluidic channel. In some implementations, the cell/plasma separation described herein, including with respect to operation 100a, may be performed while flow of the test sample through the fluidic channel is stopped or arrested in the fluidic channel. In some implementations, the lysing described herein, including with respect to operation 100d, may be performed while flow of the test sample through the fluidic channel stopped or arrested in the fluidic channel. Stopping or arresting the flow of the fluid may be performed by controlling, using the control system, a pump or other mechanisms enabling the flow to cease operation after the fluidic channel has been filled with test sample or after a predetermined amount of the test sample is in the fluidic channel. In an example, the flow cell may be filled with test sample and then flow arrested to enable cell/plasma separation and lysing of the test sample as described herein.

Process 100 may also be performed using components of other systems such as, but not limited to, those described in U.S. Patent Publication No. 2016/0202237 (Zeng), which was published on July 13, 2016, and which is incorporated herein by reference, and in U.S. Patent Publication No. 2022/0113297 (Bosy), which was published on April 14, 2022, and which is incorporated herein by reference. For example, lysing and detection of the type described herein may be performed after the separation of blood cells and plasma in the systems of U.S. Patent Publication Nos. 2016/0202237 or 2022/0113297.

In some implementations, more than one acoustic transducer (e.g., multiple acoustic transducers) may be configured and controlled in the manner of acoustic transducer 32 to perform separation and lysing on a single flow channel, such as channel 11. For example, one acoustic transducer may be located at a first region of the channel (e.g., region 17) and another acoustic transducer may be located at a second region of the channel (e.g., region 19) that is different than the first region. In some implementations, a first transducer can be used to implement separation, while a second transducer can be used to implement lysing. In some implementations, multiple acoustic transducers may be used in place of a single acoustic transducer and those multiple acoustic transducers may be configured and controlled to operate together to implement both cell/plasma separation and lysing.

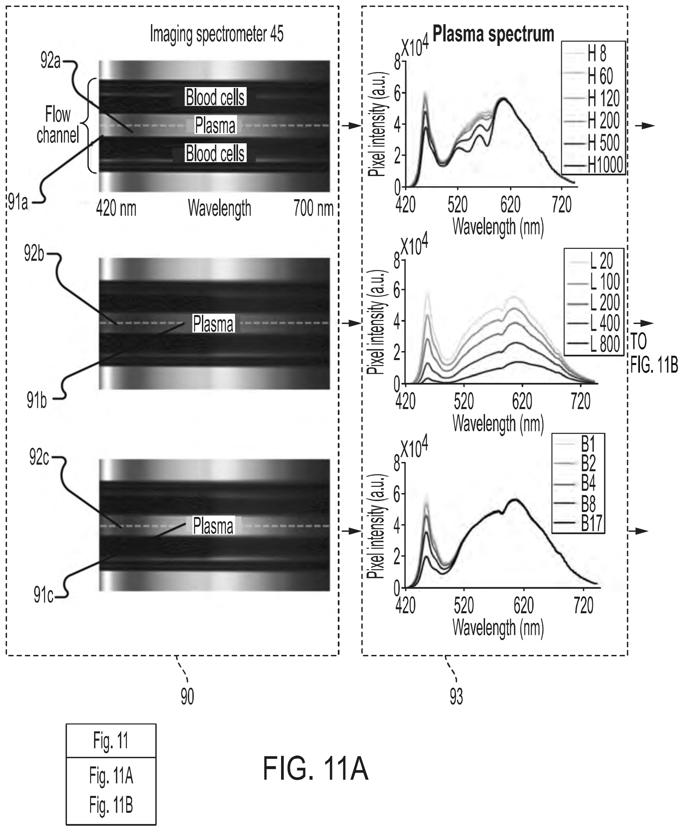

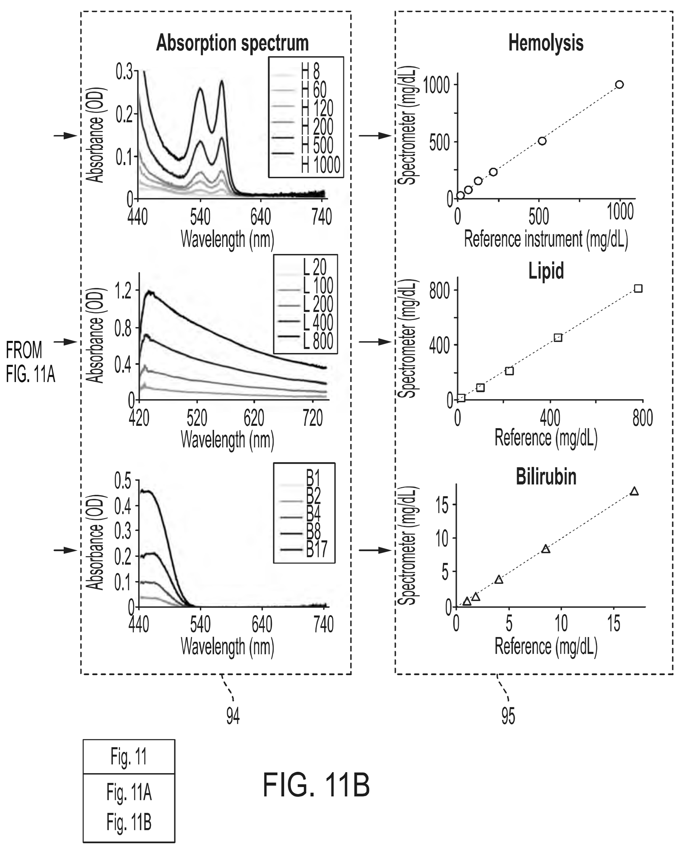

Referring to Fig. 11, images 90 on the left column thereof are example images from an imaging spectrometer, showing a fluidic channel and three different whole blood samples, with a plasma windows 91a, 91b, 91c between red blood cell layers. Since the image reflects a vertical line of light passing through a slit and dispersed by a grating, the X axis indicates wavelength (e.g., the separated blue, green, red light in the visible range). Performing optical detection across the dashed line 92a, 92b, 92c across the plasma returns a respective spectrum 93 of the plasma.

For the plasma spectrum plot in the second column, each plot shows plasma spectra from multiple whole blood samples. In this example, the blood samples had different amount of hemolysis (ranging from 8 to1000 mg/dL (milligrams per deciliter), marked as H), intralipid (20 to 800 mg/dL, marked as L), or clinical bilirubin (1 to 17 mg/dL, marked as B) spiked into the sample and mixed well, prior to the measurement.

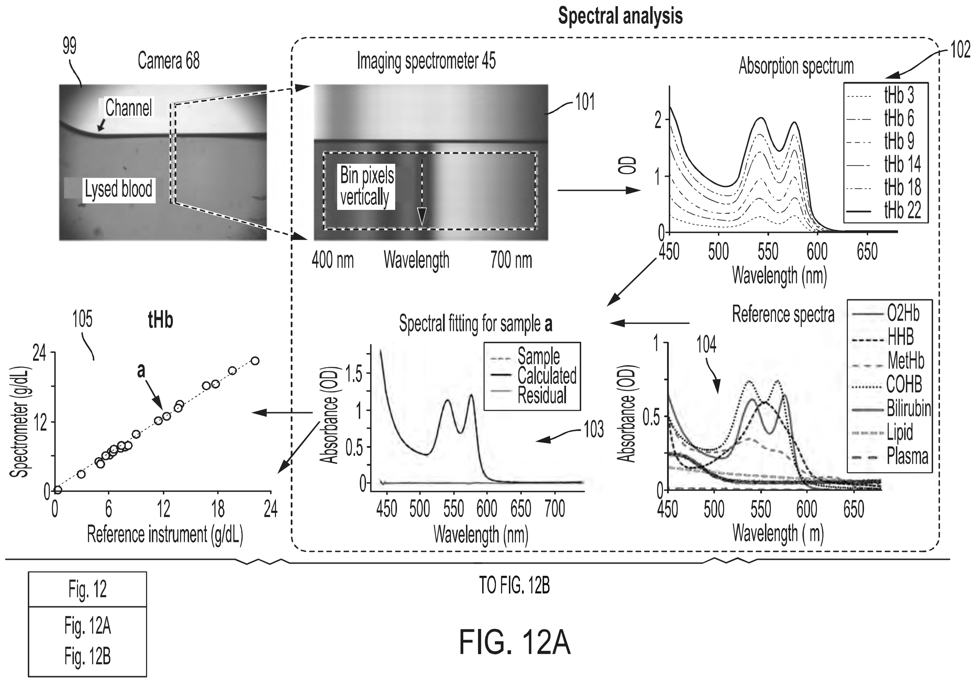

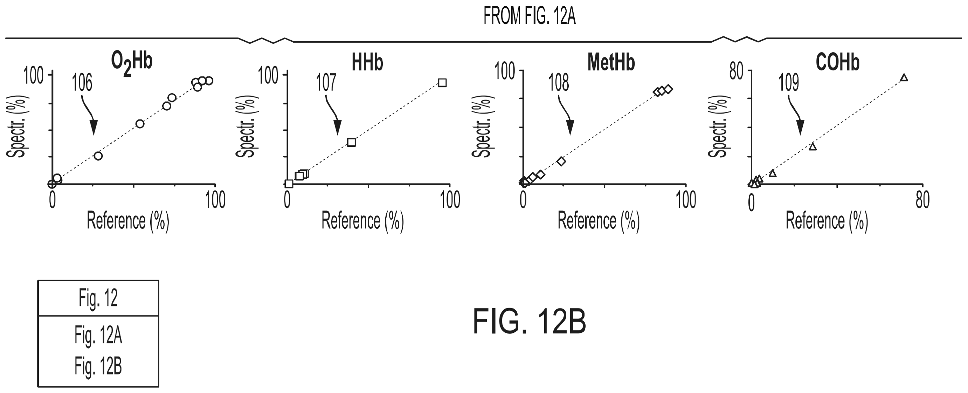

The analyte concentration can be obtained by determining the respective absorbance (OD) 94 of a specific wavelength (e.g. 570 nm for hemolysis, 610 nm for lipid, and 460 nm for bilirubin), or performing a spectral fitting using the plasma absorption spectrum and the reference spectrum of each analytes, to get the weight of each analyte. Fig. 12, described below, shows the reference spectra (O₂Hb, lipid and bilirubin spectra were used to measure hemolysis, lipid and bilirubin), and provides more details of the spectral fitting process.

The latter spectral fitting method was used to obtain respective results 95 in the fourth column for each corresponding absorbance spectrum to avoid interference between hemolysis, lipid, bilirubin and other analytes. The results 95 contain data plots showing the imaging spectrometer measured hemolysis, lipid and bilirubin, which agree with the results produced by a reference instrument.

Note that although hemolysis, lipid and bilirubin were separately spiked to different blood samples to demonstrate this spectral imaging method, all three analytes were measured from each sample image. For example, in addition to the hemolysis results shown in the top right plot, the lipid and bilirubin concentrations for each blood sample were also calculated, which are close to 0 and not shown in the plots. This multiplexed detection capability, as a strength of spectral analysis, is demonstrated in data Fig. 12 where seven analytes (tHb, O2Hb, HHb, MetHb, COHb, bilirubin and lipid) are measured from a single sample image.

Referring to Fig. 12, image 99 is a sample image from camera 68 of Fig. 8 (for sample quality check only), showing a flow channel and a fully lysed blood sample.

Image 101 is from an imaging spectrometer 45. Since this image 101 reflects a vertical line of light (the location is indicated with a dashed box on the camera 68 image) passing through a slit and dispersed by a grating, the X axis indicates wavelength (the separated blue, green, red light in the visible range). Plotting any horizontal line across the lysed blood region, or binning pixels from multiple lines vertically returns a spectrum of the lysed blood.

The blood absorbance can be calculated with the lysed blood spectrum and a water reference spectrum (e.g., from deionized water, saline or another reference reagent), using the Beer-Lambert law. The plot 102 shows the calculated blood absorption spectra from different blood samples. Here the blood sample hematocrit was adjusted to achieve different total hemoglobin (tHb) levels (3-22 g/dL).

A library of reference absorption spectra was acquired from pure O₂Hb, HHb, MetHb, COHb, bilirubin, lipid and blood plasma samples using the same imaging spectrometer (shown in the plots 104). The reference spectra were used to mathematically fit each blood sample absorption spectrum to calculate the weight of each analyte.

Plot 103 shows the spectral fitting of a sample absorption spectrum (called sample "a" here) with all the reference spectra, using least square fitting (a linear regression method). The weight and concentration of each analyte in sample "a" were calculated to be: tHb 12.4 g/dL (which includes 96.2% O2Hb, 0.8% HHb, 1.9% COHb and 1.1% MetHb), bilirubin 0.8 mg/dL, and lipid 0 mg/dL. This demonstrates the multiplexed detection capability of this spectral analysis approach - one spectral image allows for detection of many analytes all at once.

The calculated tHb and Hb fractions (O₂Hb, HHb, MetHb and COHb) are shown in plots 105 to 109, respectively. As the data suggested, the imaging spectrometer measured analyte concentrations all agree with a reference instrument.

All or part of the systems and processes described herein including but not limited to process 100, and their modifications may be configured and/or controlled at least in part by one or more computers using one or more computer programs tangibly embodied in one or more information carriers, such as in one or more non-transitory machine-readable storage media. A computer program can be written in any form of programming language, including compiled or interpreted languages, and it can be deployed in any form, including as a stand-alone program or as a module, part, subroutine, or other unit suitable for use in a computing environment. A computer program can be deployed to be executed on one computer or on multiple computers at one site or distributed across multiple sites and interconnected.

Actions associated with configuring or controlling the test system and processes described herein can be performed by one or more programmable processors executing one or more computer programs to control or to perform all or some of the operations described herein. All or part of the test systems and processes can be configured or controlled by special purpose logic circuitry, such as, an FPGA (field programmable gate array) and/or an ASIC (application-specific integrated circuit) or embedded microprocessor(s) localized to the instrument hardware.

Processors suitable for the execution of a computer program include, by way of example, both general and special purpose microprocessors, and any one or more processors of any kind of digital computer. Generally, a processor will receive instructions and data from a read-only storage area or a random access storage area or both. Elements of a computer include one or more processors for executing instructions and one or more storage area devices for storing instructions and data. Generally, a computer will also include, or be operatively coupled to receive data from, or transfer data to, or both, one or more machine-readable storage media, such as mass storage devices for storing data, such as magnetic, magneto-optical disks, or optical disks. Non-transitory machine-readable storage media suitable for embodying computer program instructions and data include all forms of non-volatile storage area, including by way of example, semiconductor storage area devices, such as EPROM (erasable programmable read-only memory), EEPROM (electrically erasable programmable read-only memory), and flash storage area devices; magnetic disks, such as internal hard disks or removable disks; magneto-optical disks; and CD-ROM (compact disc read-only memory) and DVD-ROM (digital versatile disc read-only memory).

In the description and claims provided herein, the adjectives "first", "second", "third", and the like do not designate priority or order unless context suggests otherwise. Instead, these adjectives may be used solely to differentiate the nouns that they modify.

Elements of different implementations described may be combined to form other implementations not specifically set forth previously. Elements may be left out of the systems described previously without adversely affecting their operation or the operation of the system in general. Furthermore, various separate elements may be combined into one or more individual elements to perform the functions described in this specification.

Other implementations not specifically described in this specification are also within the scope of the following claims.

## Claims

1. A system comprising:
a flow cell (10) containing a fluidic channel (11) configured to receive a sample, the sample comprising plasma and blood cells;
one or more acoustic transducers (32) configured to output acoustic waves to the fluidic channel (11), the one or more acoustic transducers (32) being configured to output acoustic waves in a first frequency range to separate the plasma from the blood cells in the fluidic channel (11) and to output acoustic waves in a second frequency range to lyse the blood cells in the fluidic channel (11), the first frequency range comprising higher frequencies than the second frequency range; and
a spectrometer (45) configured to perform detection on a region of the fluidic channel (11) containing the plasma separated from the blood cells and to perform detection on a region of the fluidic channel (11) where the blood cells have been lysed,
wherein, optionally, the spectrometer (45) is an imaging spectrometer.

2. The system of claim 1, further comprising:
an imaging camera to capture an image of the fluidic channel (11).

3. The system of claim 1, wherein the one or more acoustic transducers (32) are bound to the flow cell

4. The system of claim 1, wherein the system comprises:
a test instrument comprising the spectrometer (45), the test instrument also comprising a slot; and
a cartridge comprising the flow cell (10), the cartridge being configured to move into, and out of, the slot of the test instrument.

5. The system of claim 4, wherein the cartridge further comprises:
the one or more acoustic transducers (32) and the one or more acoustic transducers (32) are bound to the flow cell (10) or
the one or more acoustic transducers (32) are configured to move to into physical coupling with the flow cell when the cartridge is fully in the slot of the test instrument.

6. The system of claim 1, wherein the one or more acoustic transducers (32) comprise a single acoustic transducer configured to output acoustic waves in both the first frequency range and the second frequency range.

7. The system of claim 1, wherein the first frequency range is in a range of 0.5 to 30 megahertz or greater and the second frequency range is in a range of 10 kilohertz to 500 kilohertz.

8. The system of claim 1, wherein the region of the fluidic channel (11) where the plasma separated from the blood cells is detected and the region of the fluidic channel (11) where lysing is detected are different parts of the fluidic channel (11).

9. The system of claim 1, wherein the fluidic channel (11) has a first dimension in a first region and a second dimension in a second region, the second dimension being different from the first dimension, and, optionally, wherein the first dimension and the second dimension comprise channel widths or channel depths and, optionally, wherein the fluidic channel (11) comprises a curvature connecting the first region and the second region.

10. The system of claim 1, wherein an interior of the flow cell (10) defining the fluidic channel (11) comprises structures that extend from or into a surface of the interior, the structures having a height or depth within a range of 20 nanometers to 10,000 nanometers, and, optionally, wherein the structures have a shape that is at least one of: pyramidal, cylindrical, rectangular, diamond, half-spherical, conical, flat-bottom tear-drop, and spiked.

11. A method comprising:
controlling one or more acoustic transducers (32) to output acoustic waves to a fluidic channel (11) containing a sample comprised of plasma and blood cells, the one or more acoustic transducers (32) being controlled to output the acoustic waves in a first frequency range to separate the plasma from the blood cells in the fluidic channel (11);
obtaining information from a region of the fluidic channel (11) containing the plasma separated from the blood cells;
obtaining a hemolysis measurement for the region of the fluidic channel (11) containing the plasma separated from the blood cells based on the information obtained by an imaging spectrometer (45) from the region of the fluidic channel (11) containing the plasma separated from the blood cells;
controlling the one or more acoustic transducers (32) to output acoustic waves to the fluidic channel (11) after separating the plasma from the blood, the one or more acoustic transducers (32) being controlled to output acoustic waves in a second frequency range to lyse the blood cells in the fluidic channel (11), the second frequency range comprising lower frequencies than the first frequency range;
obtaining information from a region of the fluidic channel (11) where the blood cells have been lysed; and
obtaining a co-oximetry measurement for the region of the fluidic channel (11) where the blood cells have been lysed based on the information obtained by the imaging spectrometer (45) from the region of the fluidic channel (11) where the blood cells have been lysed.

12. The system of claim 11, wherein obtaining the information from the region of the fluidic channel (11) containing the plasma comprises controlling an imaging system to obtain the information from the region of the fluidic channel (11) containing the plasma; and
wherein obtaining the information from the region of the fluidic channel (11) where the blood cells have been lysed comprises controlling an imaging system to obtain the information from the region of the fluidic channel (11) where the blood cells have been lysed.

13. The method of claim 11, wherein the one or more acoustic transducers (32) are bound to a flow cell (10) containing the fluidic channel (11).

14. The method of claim 11, wherein a test instrument comprises the one or more acoustic transducers (32) and the imaging spectrometer (45), the test instrument also comprising a slot;
wherein a cartridge comprises a flow cell (10) containing the fluidic channel (11); and
wherein the method further comprises:
detecting full insertion of the cartridge into the slot; and
controlling the one or more acoustic transducers (32) to move toward the flow cell (10) after full insertion of the cartridge into the slot.

15. The method of claim 11, wherein the region of the fluidic channel (11) from which information is obtained about separation and the region of the fluidic channel (11) from which information is obtained about lysing are different parts of the fluidic channel (11).
